# EUROPEAN PATENT APPLICATION

(11) **EP 2 554 148 A1**
(43) Date of publication of application: **06.02.2013**
(21) Application number: 11759617.1
(22) Date of filing: 25.03.2011
(51) Int. Cl.: A61F 13/49, A61F 5/451, A61F 13/15, A61F 13/53

(54) **URINE ABSORPTION STRUCTURE**

(30) Priority: 26.03.2010 JP 2010073668
(71) Applicant: Unicharm Corporation, Ehime 799-0111 (JP)
(72) Inventor: WADA, Ichiro, Kanonji-shi Kagawa 769-1602 (JP); SUZUKI, Miou, Kanonji-shi Kagawa 769-1602 (JP); MURAKAMI, Hiroko, Kanonji-shi Kagawa 769-1602 (JP); FUJIOKA, Yoshihisa, Kanonji-shi Kagawa 769-1602 (JP)
(74) Representative: Eke, Philippa Dianne
(86) International application number: PCT/JP2011/057460
(87) International publication number: WO 2011/118813

(57) **Abstract**

A urine absorbent structure improved so as to prevent urine once having been absorbed by a pad from flowing back under the effect of the wearer's body weight exerted on the pad. A urine absorbent pad 10 of a urine absorbent structure includes a core 23 formed of an aggregate of water-absorbent materials. The core 23 contains, as water-absorbent materials, at least fluff pulp 23a and superabsorbent polymer particles 23b.

## Description

### {Technical Field}

This invention relates to urine absorbent structures and more specifically to urine absorbent structures suitable for automatically aspirating urine discharged by, for example, bedridden patients.

### {Background}

Conventionally, urine absorbent structures adapted for automatic aspiration of urine discharged by bedridden patients or the like are known.

For example, the urine receiver disclosed in JP 2004-267517 A (PTL 1) is connected with a vacuum pump and includes a urine absorbent material wrapped with a liquid-pervious upper sheet. The urine absorbent material is connected with a conduit tube, usually under the effect of a vacuum, so that urine having been absorbed by the urine absorbent material may be guided by the conduit tube into a urine collecting reservoir. The urine absorbent material includes hydrophilic fibers or the like and/or a water-absorbent spongy material in the upper layer and a hydrophilic foamed material and/or a suitable void structure in the lower layer. In this urine receiver, in order to prevent back-flow of urine once absorbed by the urine absorbent material from the lower layer toward the upper layer, the water absorbing rate of the urine absorbent material is set so as to be higher in the lower layer than in the upper layer.

The fluid aspiration system for the management of urinary incontinence disclosed in US 4,747,166 B2 (PTL 2) includes a urine absorbent pad and a tube connected to a vacuum source. The urine, having been absorbed by the urine absorbent pad, flows via a tube into a urine collecting container under the effect of vacuum. In this system, as the urine absorbent material, at least one of fluff pulp, a tissue laminate, a cellulosic core including open cells and the like, is used.

### {Citation List}

### {Patent Literature}

{PTL 1}: JP 2004-267517 A
{PTL 2}: US 4,747,166 B2

### {Summary}

### {Technical Problem}

In such urine absorbent structures designed such that urine is absorbed by a urine absorbent pad containing water-absorbent fibers such as fluff pulp first, and the urine is then aspirated into the reservoir under the effect of vacuum, some amount of urine might stay in the pad even after the step of such aspiration. If the wearer's body weight is exerted on the pad in such a state, the urine staying in the pad might flow back toward the wearer's skin and wet the wearer's skin so as to provide a feeling of discomfort.

An object of the present invention is to provide a urine absorbent structure improved so as to prevent urine once having been absorbed by the pad from flowing back under the effect of the wearer's body weight exerted on the pad.

### {Solution to Problem}

Some embodiments of the present invention provide a urine absorbent structure having a length direction, a width direction and a thickness direction, being orthogonal to one another. The urine absorbent article includes a urine absorbent pad and a urine collecting cup. The urine absorbent pad includes an aggregate of water-absorbent materials and inner and outer surfaces opposed to each other in the thickness direction. The urine collecting cup is located on the outer surface of the urine absorbent pad and is configured to be connectable with a vacuum suction source.

In this invention, the aggregate of water-absorbent materials contains at least fluff pulp and superabsorbent polymer particles.

According to one embodiment of this invention, the aggregate of water-absorbent materials is covered with a liquid-pervious inner sheet on the side of the inner surface and covered with a liquid-impervious outer sheet on the side of the outer surface, the outer sheet being formed with an opening in communication with the aggregate of water-absorbent materials, and the urine collecting cup is attached to the outer sheet along a peripheral edge of the opening.

According to another embodiment of this invention, the aggregate of water-absorbent materials is formed with a through-hole in communication with the opening and extending in the thickness direction, and wherein the through-hole is covered with the inner sheet.

According to even another embodiment of this invention, the aggregate of water-absorbent materials has a layered structure composed of an inner layer lying on the side of the inner surface and an outer layer lying on the side of the outer surface, and a first through-hole and a second through-hole formed in the inner layer and the outer layer, respectively, are contiguous to each other in the thickness direction to define the through-hole.

According to yet another embodiment of this invention, the through-hole has a dimension in the width direction being gradually reduced in a direction extending from the inner surface toward the outer surface.

According to still another embodiment of this invention, the dimension of the through-hole in the width direction is larger in the inner layer than in the outer layer.

According to a further embodiment of this invention, superabsorbent polymer particles are contained in the inner layer alone.

### {Advantageous Effects of Invention}

The urine absorbent pad included in the urine absorbent structure according to this invention contains at least fluff pulp and particulate or fibrous super absorbent polymer particles. Urine, having been absorbed by the superabsorbent polymer having a water retention performance higher than that of fluff pulp, does not readily leave the superabsorbent polymer and, in consequence, an uncomfortable feeling which might be experienced by the wearer of the urine absorbent structure due to backflow of urine can be alleviated.

### {Brief Description of Drawings}

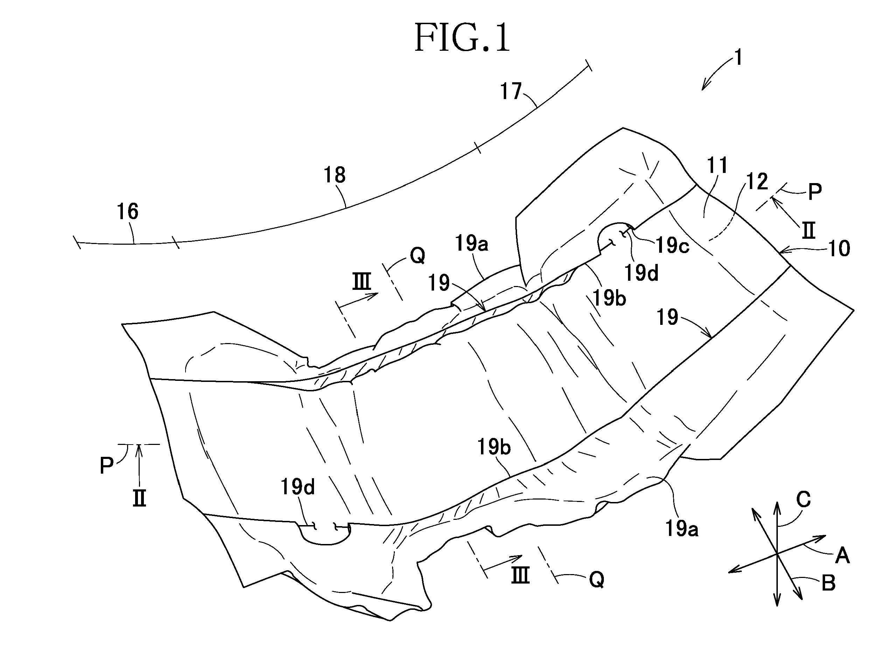

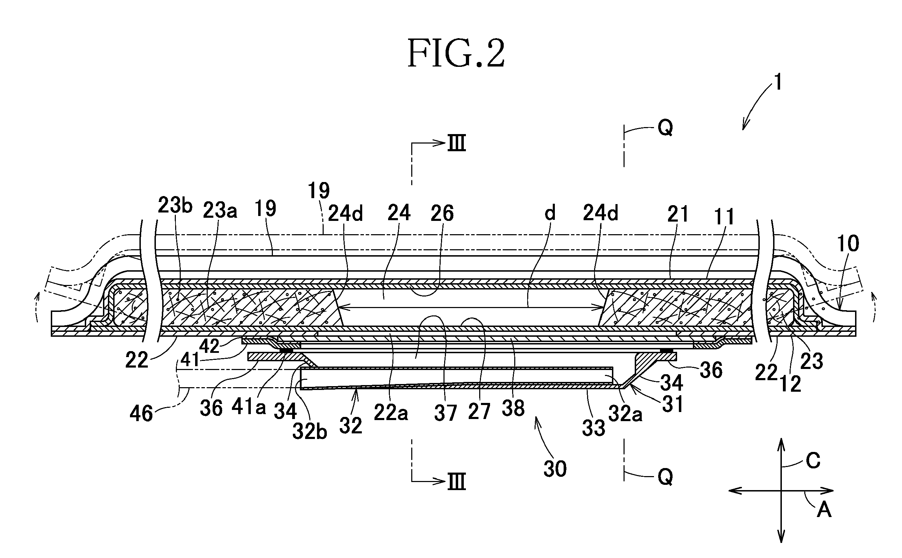

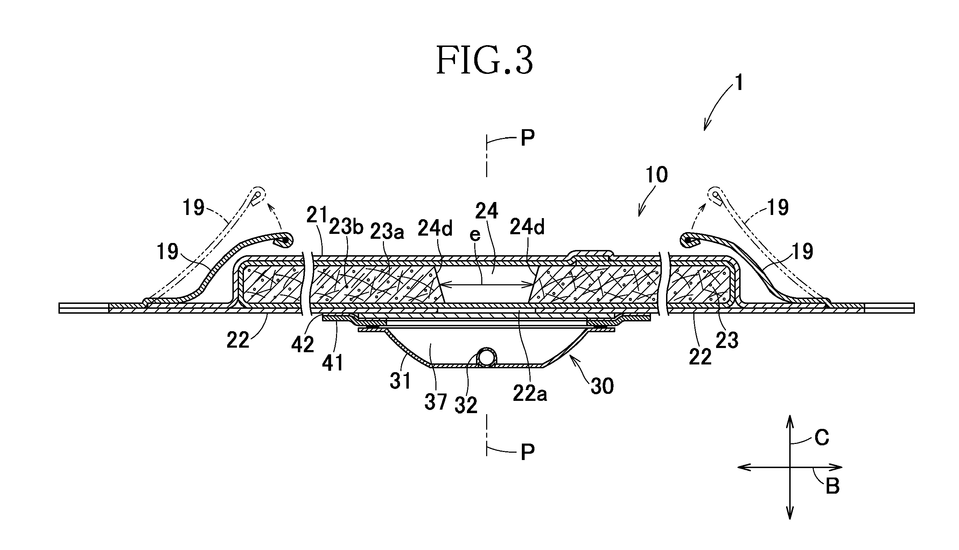

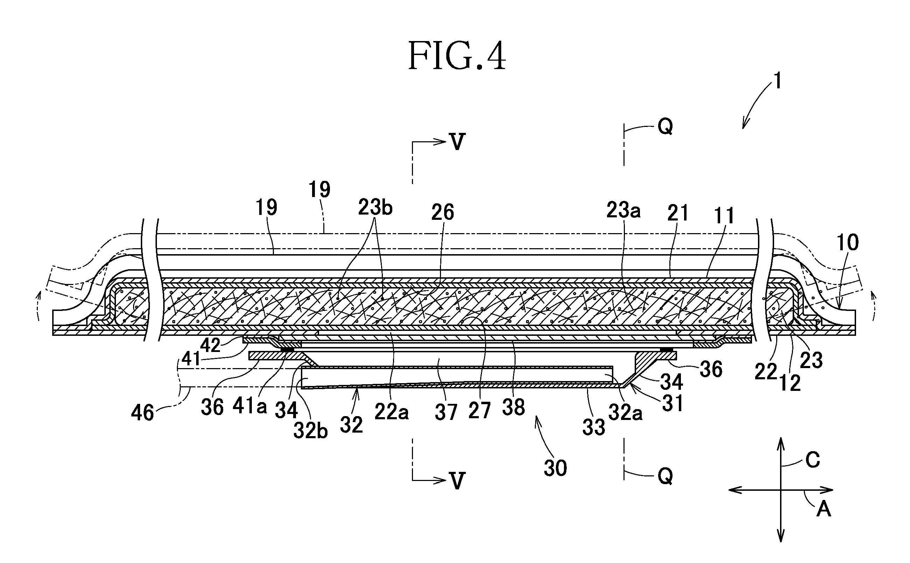

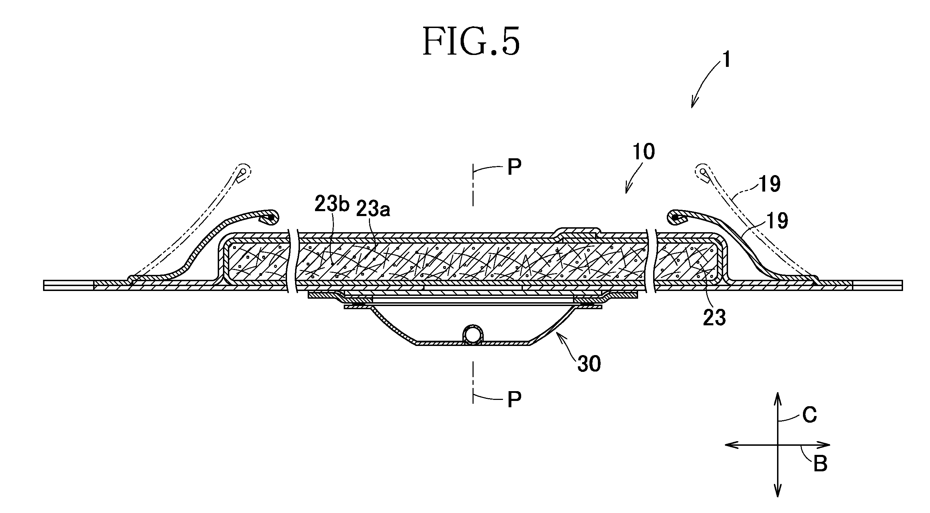

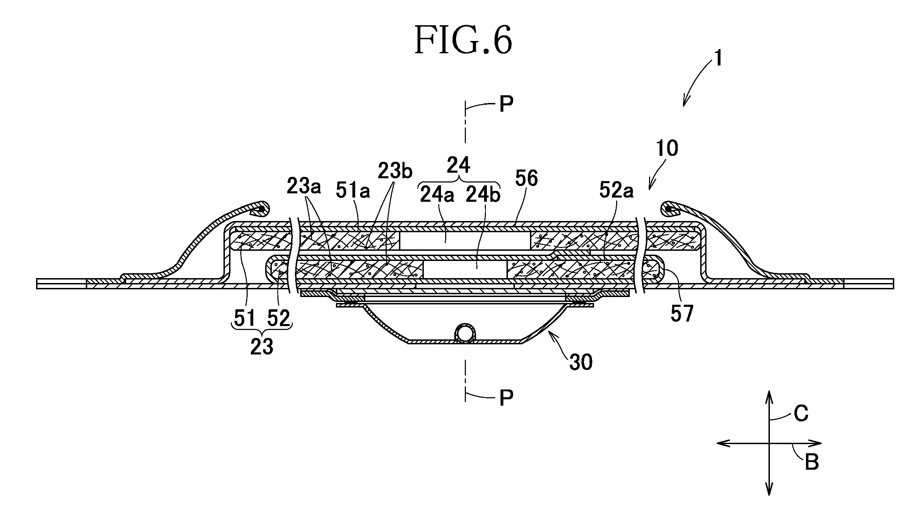

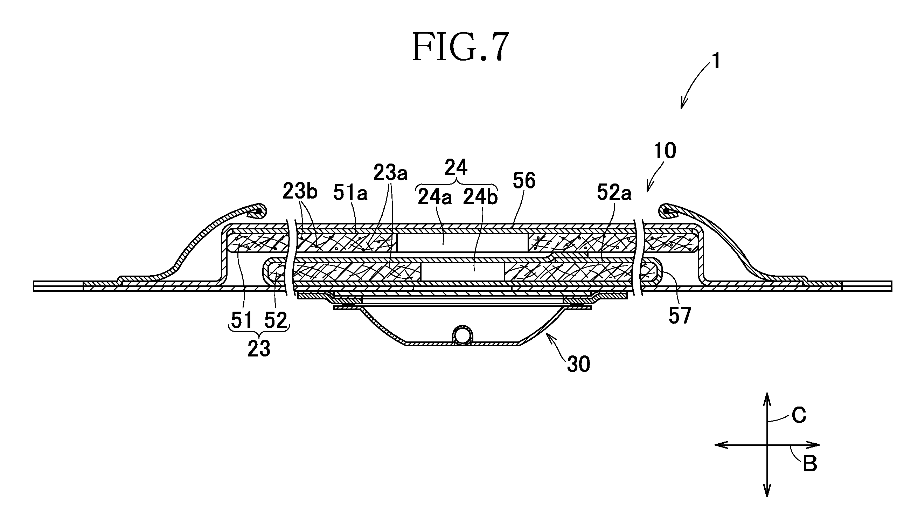

{Fig. 1} Fig. 1 is a partially cutaway perspective view of a urine absorbent structure.
{Fig. 2} Fig. 2 is a sectional view taken along line II-II in Fig. 1.
{Fig. 3} Fig. 3 is a sectional view taken along line III-III in Fig. 1.
{Fig. 4} Fig. 4 is a sectional view similar to Fig. 2, illustrating an embodiment.
{Fig. 5} Fig. 5 is a sectional view taken along line V-V in Fig. 4.
{Fig. 6} Fig. 6 is a sectional view similar to Fig. 3, illustrating another embodiment.
{Fig. 7} Fig. 7 is a sectional view similar to Fig. 6, illustrating even another embodiment.

### {Description of Embodiments}

Embodiments of a urine absorbent structure according to this invention will be described hereinafter with reference to the accompanying drawings.

Fig. 1 is a partially cutaway perspective view illustrating a pad assembly 1 as one example of the urine absorbent structure. The pad assembly 1 has a length direction A, a width direction B and a thickness direction C which are orthogonal to one another and includes a urine absorbent pad 10. The urine absorbent pad 1 has a front region 16, a rear region 17 and an intermediate region 18 wherein the front region 16 and the rear region 17 are dimensioned to be wider than the intermediate region 18 in the width direction B. The urine absorbent pad 10 further includes an inner surface 11 adapted to be put in contact with skin of the wearer (not shown) of the pad assembly 1 and an outer surface 12 adapted to be put in contact with the wearer's garment, wherein these inner and outer surfaces 11, 12 are opposite to each other in the thickness direction C. Respective opposite sides in the width direction B of the inner surface 11 are formed with barrier flaps 19. Each of the barrier flaps 19 has a distal edge 19a permanently bonded to the inner surface 11 and a proximal edge 19b not bonded to the inner surface 11, wherein the proximal edge 19b is formed with a sleeve 19c extending in the length direction A. An elastic member 19d is contractibly attached to inner wall of each sleeve 19c under tension. In this regard, the elastic member 19d is illustrated in Fig. 1 in its slightly contracted state and, in consequence, the pad assembly 1 is illustrated to be curved in the length direction A and to have the outer surface 12 being outwardly convex. Such pad assembly 1 is further curved in the length direction A and the inner surface 11 is put in contact with the wearer's skin so that the front region 16 may define a front waist region, the rear region 17 may define a rear waist region and the intermediate region 18 may define a crotch region. After putting the pad assembly 1 on the wearer's skin, a diaper, a diaper cover, pants or the like is put on the wearer's body from the outside of the pad assembly to set the pad assembly 1 substantially in a state kept contacted to the wearer's skin. In the pad assembly 1 having its inner surface 11 put in contact with the wearer's skin, the elastic members 19d further contract so that the proximal edges 19b of the respective barrier flaps 19 rise from the inner surface 11 and come in contact with the wearer's skin. The pad assembly 1 and the urine absorbent pad 10 defining part thereof are shaped symmetrically about a longitudinal center line P-P bisecting respective dimensions of them as measured in the width direction B.

Fig. 2 is a sectional view taken along line II-II in Fig. 1, wherein the pad assembly 1 is under tension in the length direction A as well as in the width direction B, the elastic members 19d are also under tension in the length direction A and the urine absorbent pad 10 is in a horizontal posture. In Fig. 2, the pad assembly 1 is illustrated as partially cutaway. Line II-II is the line corresponding to the longitudinal center line P-P. Referring to Fig. 2, the urine absorbent pad 10 includes a liquid-pervious upper sheet 21 defining the inner surface 11, a lower sheet 22 defining the outer surface 12 and an aggregate of water-absorbent materials forming a core 23 interposed between the upper sheet 21 and the lower sheet 22. The upper sheet 21 and the lower sheet 22 extend outward in the length direction A beyond the core 23 and are put flat and joined together along the respective extensions by fusion bonding with use of heat-embossing or by bonding with use of adhesive. The core 23 is formed with a through-hole 24 extending through the core 23 in the thickness direction C at a position anterior to a transverse center line Q-Q bisecting a dimension as seen in the length direction A. In this through-hole 24, a pair of peripheral wall segments 24d opposed to each other in the length direction A are tapered downward so that a distance between these peripheral wall segments 24d is gradually reduced from the inner surface 11 toward the outer surface 12. The core 23 is wrapped with the inner side tissue paper sheet 26 and the outer side tissue paper sheet 27, and the through-hole 24 also is covered with these tissue paper sheets 26, 27. The barrier flaps 19 attached to the inner surface 11 of the urine absorbent pad 10 and having risen from the inner surface 11 under contraction of the elastic members 19d are indicated by imaginary lines. Such a urine absorbent pad 10 is provided with a urine collecting cup 30 attached to the outer surface 12 just below the through-hole 24. The urine collecting cup 30 includes a cup body 31 and a conduit 32.

The cup body 31 includes a bottom 33, a peripheral wall 34, a flange 36 extending outward from the peripheral wall 34 in the length direction and an opening 37 defined by the peripheral wall 34 so as to be in fluid-communication with the through-hole 24 of the core 23. A plastic film 41 for sealing is attached to the flange 36 with an adhesive 41a or by fusion bond and the plastic film 41 is bonded to the outer sheet 22 around an opening 22a formed in the outer sheet 22 with adhesive 42 applied to the plastic film 41. Immediately below the opening 22a, an breathable and liquid-pervious topsheet 38 is provided to cover the opening 37 of the cup body 31. The topsheet 38 is bonded to the flange 36 of the cup body 31 with the adhesive 41a and the adhesive 42. A portion of the plastic film 41 extends outward beyond the periphery of the topsheet 38 in the length direction A and the width direction B is bonded to the outer sheet 22.

The conduit 32 in the urine collecting cup 30 extends in the length direction A through the peripheral wall 34 of the cup body 31 and has an inner side opening 32a inside the cup body 31 and an outer side opening 32b outside the cup body 31. A vacuum pipe 46 indicated by an imaginary line extending from a vacuum suction source (not shown) such as a vacuum pump is detachably connected to the outer side opening 32b. In order that the vacuum provided with the vacuum pipe 46 and the conduit 32 may rapidly act on the cup body 31 and the inner side of the urine absorbent pad 10, the urine collecting cup 30 is attached to the outer sheet 22 of the urine collecting pad 10 in an air-tight fashion.

Fig. 3 is a sectional view taken along line III-III in Fig. 1. In the urine absorbent pad 10, the core 23 is formed in its middle as viewed in the width dimension B with the through-hole 24 and a spacing dimension e between a pair of tapered peripheral wall segments 24d opposed to each other in the width direction B and defining the through-hole 24 is gradually reduced from the inner surface 11 toward the outer surface 12. The inner sheet 21 and the outer sheet 22 extend outward in the width direction B beyond the side edges of the core 23 and are put flat and bonded together along respective extensions of these sheets 21, 22 with a hot melt adhesive (not shown). In the urine collecting cup 30, the cup body 31 and the conduit 32 are integrally formed wherein the conduit 32 lies on the longitudinal center line P-P.

In the pad assembly 1, the core 23 of the urine absorbent pad 10 contains at least one of fluff pulp 23a, other water-absorptive natural fibers and semisynthetic fibers, as constituents having relatively high water absorption rate. The core 23 contains, in addition, particulate and/or fibrous superabsorbent polymer having a relatively high water retention performance, more preferably, superabsorbent polymer particles 23b. In a specific embodiment, the core 23 is formed of an aggregate of water-absorbent materials which contains at least fluff pulp 23a and superabsorbent polymer particles 23b. While not particularly specified, fluff pulp in the core 23 may be, for example, in a range of 300 to 800 g/m² on the assumption that the pad assembly 1 is for adults having a body weight in a range of 30 to 70 kg. Superabsorbent polymer particles 23b to be used may have a water retention performance higher than that of fluff pulp 23a. Examples of such superabsorbent polymer particles 23b include starch acrylonitrile copolymer, starch acrylic acid graft copolymer and polyvinyl alcohol. The amount of superabsorbent polymer particles 23b to be used is preferably equivalent to 10 to 100% of the amount of fluff pulp 23a to be used. In this regard, the superabsorbent polymer particles 23b may be partially replaced by fibrous superabsorbent polymer.

While the size of the through-hole 24 may be appropriately adjusted depending on the size of the urine absorbent pad 10, this through-hole 24 may be dimensioned to be in a range of 50 to 200 mm in the length direction A and in a range of 10 to 30 mm in the width direction, for example, assuming that the pad assembly 1 is for adult having body weight in a range of 30 to 70 kg. The taper angle of the peripheral wall 24d defining the through-hole 24 is preferably in a range of 3 to 45° with respect to the perpendicular line extending in parallel to the transverse center line Q-Q in Fig. 2 or the longitudinal center line P-P in Fig. 3. At least one of the tissue paper sheets 26, 27 wrapping the core 23 may be replaced by a liquid-pervious nonwoven fabric formed of thermoplastic synthetic fibers. As material of the inner sheet 21, at least one of a liquid-pervious nonwoven fabric formed of thermoplastic synthetic fibers and a liquid-pervious porous plastic film may be used. As material of the outer sheet 22, for example, a liquid-impervious plastic film, a liquid-impervious nonwoven fabric formed of thermoplastic synthetic fibers or a composite sheet composed of a liquid-impervious plastic film and a nonwoven fabric laminated on the outer surface thereof. As material of the barrier flaps 19 attached to the urine absorbent pad 10, at least one of a nonwoven fabric formed of thermoplastic synthetic fibers and a plastic film, which are preferably liquid-impervious, may be used.

The urine collecting cup 30 is obtained by injection molding a flexible elastic material such as low density polyethylene or silicon rubber so that the cup body 31 and the conduit 32 may be integrally formed. As material of the topsheet 38 for the urine collecting cup 30, for example, at least one of a breathable and liquid-pervious nonwoven fabric formed of thermoplastic synthetic fibers and a breathable and liquid-pervious mesh fabric formed of, for example, a thermoplastic synthetic resin may be used. As material of the plastic film 41 used to attach the urine collecting cup 30 to the urine absorbent pad 10, for example, at least one of a polyethylene film, a polypropylene film, a polyester film and a laminate film composed of a polyethylene film and a polyester film may be used.

The pad assembly 1 configured as has been described above may be used so as to cooperate with a sensor (not shown) incorporated therein so that this sensor may electrically detect that the inner sheet 21 and/or the core 23 is wetted with urine. For example, when a pair of electrodes is wetted with urine and assumes a conductive state, the sensor detects it and thereupon actuates a vacuum suction source such as the vacuum pump so that the inner space of the cup body 31 may be pressure-reduced via the vacuum pipe 46 (See Fig. 2). Such sensor is usually provided at an appropriate location, for example, on the surface of the inner sheet 21, between the inner sheet 21 and the core 23 or between the core 23 and the topsheet 38. The pad assembly 1 used in such a fashion will function as a part of the automatic urine absorbent apparatus including the vacuum suction source. In this regard, the pad assembly 1 may be connected, instead of using such sensor, to the vacuum suction source adapted to be manually actuated or to be actuated by a timer or the like.

When the pad assembly 1 is put on the wearer's body, the urine absorbent pad 10 is set with respect to the wearer's body so that the core 23 may be opposed to the wearer's urethral orifice, more preferably the through-hole 24 of the core 23 may face the urethral orifice. Upon urination toward the urine absorbent pad 10 set in this manner, a portion of urine flows into the through-hole 24 to wet the topsheet 38 and moves into the cup body 31. The topsheet 38 is wetted and some of the meshes and/or fiber interstices thereof are infilled with urine and enhance the vacuum within the cup body 31. In consequence, the cup body 31 intensely sucks urine. Another portion of urine flows into the core 23 . Urine having flown into the core 23 passes partially through the core 23 and the topsheet 38 under the effect of vacuum within the cup body 31 into the cup body 31 and the remaining portion of urine is absorbed by fluff pulp 23a and superabsorbent polymer particles 23b of the core 23 and stays behind in the core 23. The portion of urine transferred into the cup body 31 flows through the inner opening 32a into the conduit 32 and is guided to the outside of the pad assembly 1 via the vacuum pipe 46. When the wearer's body weight is exerted on the urine absorbent pad 10, of urine staying behind in the core, a fraction absorbed by fluff pulp 23a absorbed in fluff pulp 23a may partially flow back toward the wearer's skin but a fraction absorbed in superabsorbent polymer particles 23b having high water retention performance does not readily leave these superabsorbent polymer particles 23b. In this way, the pad assembly 1 makes it possible to reduce the amount of urine which may flow back toward the wearer's skin at least by the amount of urine absorbed in superabsorbent polymer particles 23b, and an uncomfortable feeling which might be experienced by the wearer due to back-flow of urine may be correspondingly alleviated.

Figs. 4 and 5 illustrate another embodiment wherein Fig. 4 is a view similar to Fig. 2, illustrating the pad assembly 1 according to this embodiment and Fig. 5 is a sectional view taken along line V-V in Fig. 4. The pad assembly 1 illustrated in Figs. 4 and 5 also includes the urine absorbent pad 10 and the urine collecting cup 30. The urine collecting cup 30 is similar to that illustrated by way of example in Figs. 2 and 3 but the urine absorbent pad 10 is distinguished from that illustrated in Figs. 2 and 3. Specifically, the core 23 is a mixture of fluff pulp 23a and superabsorbent polymer particles 23b as in the aforementioned embodiment but is distinguished from the aforementioned embodiment in that the core 23 is not formed with the through-hole 24 illustrated by way of example in Figs. 2 and 3. However, even in such a pad assembly 1 illustrated in Figs. 4 and 5, high urine absorbing performance of superabsorbent polymer particles 23b makes it possible to restrict a problem that urine staying behind in the core 23 might flow back toward the wearer's skin. In this regard, this pad assembly 1 may be put on the wearer's body so that the urine collecting cup 30 may face the urethral orifice of the wearer.

Fig. 6 is a view similar to Fig. 3, illustrating still another embodiment. The core 23 in the pad assembly 1 illustrated in Fig. 6 has a layer construction composed of an upper layer 51 and a lower layer 52. The upper layer 51 contains fluff pulp in an amount of 250 to 300 g/m² and superabsorbent polymer particles 23b in an amount of 90 to 150 g/m² and is formed in the middle as viewed in the width direction B with a first through-hole 24a. An upper surface 51a of the upper layer 51 is wrapped with a tissue paper sheet 56. The lower layer 52 contains fluff pulp 23a in a range of 270 to 320 g/m² adjusted so that the amount thereof may be higher than the amount in the upper layer 51 by about 20 to about 40 g/m². The lower layer 52 contains superabsorbent polymer particles 23b in an amount of 80 to 140 g/m² adjusted so that the amount thereof may be lower than the amount in the upper layer 51 by about 5 to 30 g/m². The lower layer 52 is formed in the middle as viewed in the width direction B with a second through-hole 24b which is contiguous to but narrower than the first through-hole 24a. Though not illustrated, the dimension in the length direction A of the second through-hole 24b is smaller than the dimension in the length direction A of the first through-hole 24a. For example, on the assumption that the pad assembly 1 is for adults having a body weight in a range of 30 to 70 kg, the first through-hole 24a may be formed so as to have a width of 25 +/- 3 mm and a length of 180 +/- 20 mm and the second through-hole 24b may be formed so as to have a width of 15 +/- 3 mm and a length of 65 +/- 20 mm. The through-hole 24 of the core 23 formed of the first through-hole 24a and the second through-hole 24b has an inner diameter being gradually reduced from the inner sheet 21 toward the outer sheet 22 so as to ensure the same advantageous function and effect as in the core 23 illustrated in Figs. 2 and 3, such that flow of urine from the core 23 into the cup body 31 may be facilitated but back-flow of urine from the cup body 31 toward the core 23 may be restricted. In comparison to the core in which fluff pulp and superabsorbent polymer particles are evenly mixed together, the content amount of superabsorbent polymer particles 23b is higher in the upper layer 51 than in the lower layer 52 and, in consequence, superabsorbent polymer particles 23b in the upper layer 51, swollen due to absorption of urine, may more positively prevent urine having been absorbed by the fluff pulp 23a in the lower layer 52 from flowing back toward the inner sheet 21. The entire peripheral surface inclusive of a top surface 52a is wrapped with a tissue paper sheet 57.

Fig. 7 is a view similar to Fig. 6, illustrating yet another embodiment. It should be noted that the core 23 illustrated in Fig. 7 also has the upper layer 51 and the lower layer 52 but superabsorbent polymer particles 23b are contained in the upper layer 51 only. As an example, the upper layer 51 contains fluff pulp in an amount of 250 to 300 g/m² and superabsorbent polymer particles 23b in an amount of 180 to 250 g/m². The lower layer 52 contains fluff pulp in an amount of 300 to 400 g/m². In the core 23 having such upper and lower layers 51, 52, superabsorbent polymer particles, swollen due to absorption of urine in the upper layer 51, are in a state readily forming a gel block among them and such gel block effectively functions to prevent urine having been absorbed by fluff pulp 23a in the lower layer 52 from flowing back toward the upper layer 51.

### {Reference Signs List}

- 1: pad assembly (urine absorbent structure)
- 10: urine absorbent pad
- 21: upper sheet
- 22: lower sheet
- 22a: opening
- 23: core (assembly)
- 23a: fluff pulp
- 23b: superabsorbent polymer particles
- 24: through-hole
- 30: urine collecting cup
- 51: upper layer (inner layer)
- 52: lower layer (outer layer)
- A: length direction
- B: width direction
- C: thickness direction
- e: dimension

## Claims

1. A urine absorbent structure having a length direction, a width direction and a thickness direction being orthogonal to one another, the urine absorbent structure comprising:
a urine absorbent pad including an aggregate of water-absorbent materials and inner and outer surfaces opposed to each other in the thickness direction; and
a urine collecting cup located on the outer surface of the urine absorbent pad and configured to be connectable with a vacuum suction source, wherein:
the aggregate of water-absorbent materials contains at least fluff pulp and superabsorbent polymer particles.

2. The urine absorbent structure according to claim 1, wherein the aggregate of water-absorbent materials is covered with a liquid-pervious inner sheet on the side of the inner surface and covered with a liquid-impervious outer sheet on the side of the outer surface,
the outer sheet is formed with an opening in communication with the aggregate of water-absorbent materials, and
the urine collecting cup is attached to the outer sheet along a peripheral edge of the opening.

3. The urine absorbent structure according to claim 2, wherein the aggregate of water-absorbent materials is formed with a through-hole in communication with the opening and extending in the thickness direction, and wherein
the through-hole is covered with the inner sheet.

4. The urine absorbent structure according to claim 3, wherein the aggregate of water-absorbent materials has a layered structure composed of an inner layer lying on the side of the inner surface and an outer layer lying on the side of the outer surface, and a first through-hole and a second through-hole formed in the inner layer and the outer layer, respectively, are contiguous to each other in the thickness direction to define the through-hole.

5. The urine absorbent structure according to claim 3 or 4, wherein the through-hole has a dimension in the width direction being gradually reduced in a direction extending from the inner surface toward the outer surface.

6. The urine absorbent structure according to claim 4 or 5, wherein the dimension of the through-hole in the width direction is larger in the inner layer than in the outer layer.

7. The urine absorbent structure according to any one of claims 4 through 6, wherein superabsorbent polymer particles are contained in the inner layer alone.
